# EUROPEAN PATENT SPECIFICATION

(11) **EP 3 381 936 B1**
(45) Date of publication and mention of the grant of the patent: **24.03.2021**
(21) Application number: 16868079.1
(22) Date of filing: 28.11.2016
(51) Int. Cl.: C07K 14/60, C12Q 1/68, G01N 33/574, G01N 33/74

(54) **DIAGNOSIS OF PROSTATE CANCER USING GHRELIN O-ACYLTRANSFERASE (GOAT)**
DIAGNOSE VON PROSTATAKREBS MIT GHRELIN-O-ACYLTRANSFERASE (GOAT)
DIAGNOSTIC DU CANCER DE LA PROSTATE EN UTILISANT GHRELIN-O-ACYLTRANSFERASE (GOAT)

(30) Priority: 27.11.2015 ES 201531731
(43) Date of publication of application: 03.10.2018
(73) Proprietor: Universidad De Córdoba, 14004 Córdoba (ES); Servicio Andaluz de Salud, 41071 Sevilla (ES)
(72) Inventor: CASTAÑO FUENTES, Justo P., 14004 Córdoba (ES); LUQUE HUERTAS, Raúl M., 14004 Córdoba (ES); GAHETE ORTIZ, Manuel D., 14004 Córdoba (ES); HORMAECHEA AGULLA, Daniel, 14004 Córdoba (ES); REQUENA TAPIA, María José, 41071 Sevilla (ES); GÓMEZ GÓMEZ, Enrique, 41071 Sevilla (ES); CARRASCO VALIENTE, Julia, 41071 Sevilla (ES); IBÁÑEZ COSTA, Alejandro, 14004 Córdoba (ES); MORENO, María del Mar, 41071 Sevilla (ES); VALERO ROSA, José, 41071 Sevilla (ES)
(74) Representative: Hoffmann Eitle
(86) International application number: PCT/ES2016/070844
(87) International publication number: WO 2017/089642

(56) References cited:
- EP-A2- 1 847 620
- WO-A1-02/090387
- WO-A1-2011/154570
- WO-A1-2013/155633
- HORMAECHEA-AGULLA DANIEL ET AL: "Ghrelin O-acyltransferase (GOAT) enzyme is overexpressed in prostate cancer, and its levels are associated with patient's metabolic status: Potential value as a non-invasive biomarker", CANCER LETTERS, NEW YORK, NY, US, vol. 383, no. 1, 28 September 2016 (2016-09-28), pages 125-134, XP029785564, ISSN: 0304-3835, DOI: 10.1016/J.CANLET.2016.09.022
- INGE SEIM ET AL.: 'Ghrelin O-acyltransferase (GOAT) is expressed in prostate cancer tissues and cell lines and expression is differentially regulated in vitro by ghrelin' REPRODUCTIVE BIOLOGY AND ENDOCRINOLOGY, [Online] vol. 11, no. 1, 23 July 2013, ISSN 1477-7827 page 70, XP055496551 ISSN: 1477-7827 Retrieved from the Internet: <URL:doi: 10.1186/1477-7827-11-70>

## Description

### FIELD OF THE ART

The present invention is comprised within the field of molecular biology and medicine and relates to an enzyme referred to as ghrelin-O-acyltransferase (GOAT) and the uses thereof. Specifically, it relates to the use of this enzyme for obtaining data that can be used in the diagnosis and/or detection of patients with prostate cancer.

### PRIOR STATE OF THE ART

Ghrelin is a hormone that is synthesized primarily in the stomach [1] although it is also expressed in a wide range of tissues where it acts as a paracrine or autocrine factor [2-5]. Ghrelin is the main endogenous ligand of the growth hormone secretagogue receptor (GHS-R) type 1a (GHS-R1a). The ghrelin/GHS-R1a system is now known to act in various tissues, many of them related to metabolic function regulation [6-8]. For ghrelin to bind to the GHS-R1a receptor, and to therefore act, its serine 3 (Ser3) residue must first be acylated (through n-octanoacylation). The enzyme responsible for this post-translational modification is ghrelin-O-acyl-transferase or GOAT [9, 10]. This enzyme belongs to the membrane-bound O-acyltransferase (MBOAT) superfamily [11], and is therefore also referred to as MBOAT4 [12].

The human GOAT gene (MBOAT4) is located in chromosome 12 and its sequence has a high degree of conservation among vertebrates [13]. GOAT is an integral membrane protein that octanoylates the Ser3 residue of ghrelin in the endoplasmic reticulum following elimination of the signal peptide [14]. At the mRNA level, the GOAT enzyme is expressed in a large number of tissues such as the stomach, the pancreas, the skeletal muscle, the heart, the intestine, or the bones [15]; an expression pattern that partially overlaps the pattern exhibited by ghrelin [15]. In fact, the presence of GOAT (at the mRNA and protein level) has been observed in individual ghrelin-producing cells [16]. However, some studies have demonstrated that the expression levels of GOAT do not completely correlate with the expression levels of ghrelin, but rather are more similar to the expression levels shown by a splicing variant of the ghrelin gene referred to as In1-Ghrelin [17], suggesting the possible existence of additional targets for GOAT.

It is important to indicate that the system formed by ghrelin, its splicing variant, In1-Ghrelin, the associated receptors (GHS-R1a and a truncated receptor referred to as GHS-R1b), and the GOAT enzyme may perform a relevant regulatory role in several types of tumor pathologies. In fact, all the components of this regulatory system were found to be expressed in gastric tumors [18-20], and/or intestinal tumors [21], and/or carcinoid tumors of the pancreas [20, 22], as well as in breast cancer [23] and prostate cancer [24], although their expression levels depend on the detection method used [18-21].

However, the authors of the present invention are the first to suggest and provide experimental data supporting the determination of one of the specific components of the system, particularly the GOAT enzyme, as a tool that can be used clinically in the clinical diagnosis of prostate cancer. WO02/090387 describes other biomarkers of prostate cancer.

### BRIEF DESCRIPTION OF THE INVENTION

A first aspect of the present invention refers to a method for the diagnosis of prostate cancer in a human subject, wherein the method comprises:
a. detecting and quantifying, *in vitro,* the expression of ghrelin-O-acyltransferase (GOAT) enzyme mRNA in a prostate tissue biopsy or sample isolated from said subject or the amount of the GOAT protein in a blood, plasma, or serum sample isolated from said subject;
b. comparing the levels obtained in step (a) with a reference level or with the levels obtained in a control sample obtained from a healthy human subject; and
c. assigning the subjects having increased levels with respect to a reference level to the group of individuals suffering from prostate cancer,

In a particular embodiment of the present invention, step c) comprises:
c. assigning the subjects having levels increased by at least 2-fold, with respect to the levels obtained in a control sample obtained from a healthy human subject, to the group of individuals suffering from prostate cancer.

Another particular embodiment of the present invention refers to the *vitro* use of a kit or device in the method of the first aspect of the invention, wherein the kit or device comprises specific primers for the determination of the expression levels of GOAT mRNA by means of PCR, wherein preferably the kit or device comprises primers consisting essentially of sequences SEQ ID NO 1 and SEQ ID NO 2. More preferably, the kit or device further comprises at least one or any combination of the following reagents: DNA polymerase, dNTPs, MgCl₂, markers, stabilizers, DNAses, and the reverse transcriptase (RT) enzyme.

Another particular embodiment of the present invention refers to the *vitro* use of a kit or device in the above method, wherein the kit or device comprises antibodies or fragments thereof that are specific against the GOAT protein.

### SUMMARY OF OTHER ASPECTS OF THE DISCLOSURE

The problem addressed by the authors of the present invention relates to providing prostate cancer diagnostic tools. In particular, the problem addressed by them relates to providing a diagnostic tool which serves as a clinically efficient alternative to the use of the prostate-specific antigen (PSA).

In addressing this problem, the authors have found that the use of the levels of the amount or concentration of the ghrelin-O-acyltransferase (GOAT) enzyme or of the mRNA encoding said protein, obtained ex *vivo* or *in vitro* from an isolated human biological sample, constitutes a tool that is efficient as a biomarker or indicator for obtaining data that can be used in the clinical diagnosis of prostate cancer. In particular, the authors of the present invention have observed how plasma GOAT levels can distinguish patients with prostate cancer from controls (healthy individuals) with a high specificity and sensitivity of 66% and 81%, respectively; which levels demonstrate being a diagnostic tool that can be used clinically.

### BRIEF DESCRIPTION OF THE DRAWINGS

**Figure 1****.** Expression levels of GOAT in biopsy samples from patients with prostate cancer and controls (healthy individuals). The copy number adjusted by a normalization factor of n=37 tumor cases and n=10 controls is depicted. The ROC curve showing that the expression levels of GOAT in biopsy samples can distinguish patients with prostate cancer from controls (healthy individuals).
**Figure 2****.** Levels of GOAT in plasma samples from patients with prostate cancer and controls (healthy individuals). The concentration of GOAT (ng/mL) of n=85 tumor cases and n=29 controls is depicted. The ROC curve showing that the plasma GOAT levels can distinguish patients with prostate cancer from controls (healthy individuals) with a high specificity and sensitivity.
**Figure 3****.** Correlations of plasma GOAT levels with the levels of other tumor markers such as total PSA (TPSA), free PSA (FPSA), ca153, and cifra_211 in patients with prostate cancer.
**Figure 4****.** Correlation of plasma GOAT levels with plasma glycosylated hemoglobin levels in patients with prostate cancer.
**Figure 5****.** Correlations of plasma GOAT levels with glucose levels and the HOMA-IR index in patients with prostate cancer who have a metabolic syndrome.

### DETAILED DESCRIPTION OF THE INVENTION

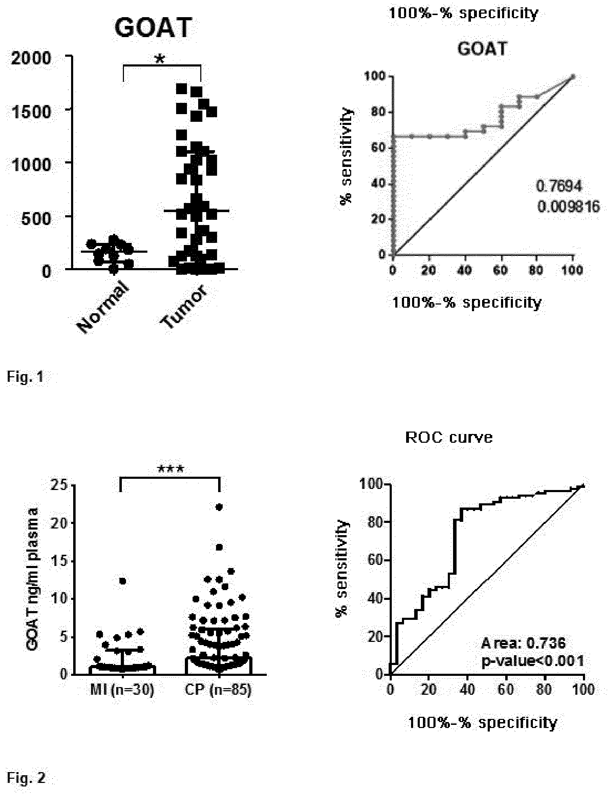

The results shown in the present invention demonstrate that the expression levels of GOAT, measured by means of real-time quantitative PCR (qPCR), are significantly increased in samples originating from biopsies (samples isolated from prostate tissue) of patients with prostate cancer in comparison with samples originating from the biopsies of healthy patients (in which the biopsy was negative for the presence of prostate cancer) (Figure 1, left panel). Furthermore, as shown in the ROC curve shown in Figure 1 (right panel), the expression levels of GOAT in said biopsied samples can significantly distinguish patients with prostate cancer from healthy individuals with a high sensitivity and specificity.

On the other hand, the results shown herein also indicate that, although the urine GOAT protein levels do not seem to be high enough for them to be used for the diagnosis of prostate cancer, measurement of the plasma GOAT protein levels allows distinguishing patients with prostate cancer from controls (healthy individuals) with a high specificity and sensitivity (Figure 2, left panel). Furthermore, the data that is shown demonstrate that the measurement of this marker in blood samples, particularly in plasma, results in a diagnostic tool that is particularly clinically efficient. So as shown in the ROC curve of Figure 2 (right panel), a plasma GOAT value of 1.212 ng/mL shows a sensitivity of 81% and a specificity of 67% for distinguishing patients with prostate cancer from those without.

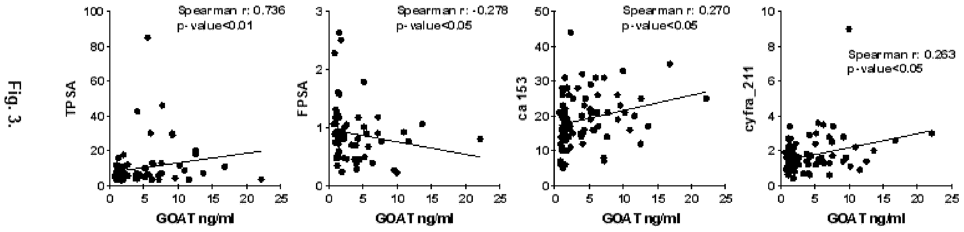

Furthermore, the plasma GOAT levels detected in patients with prostate cancer correlated positively with total PSA (TPSA) levels and negatively with free PSA (FPSA) levels (Figure 3). Similarly, the plasma GOAT levels detected in patients with prostate cancer also correlated positively with other two tumor markers such as ca1 5.3 and cyfra_211 (Figure 3).

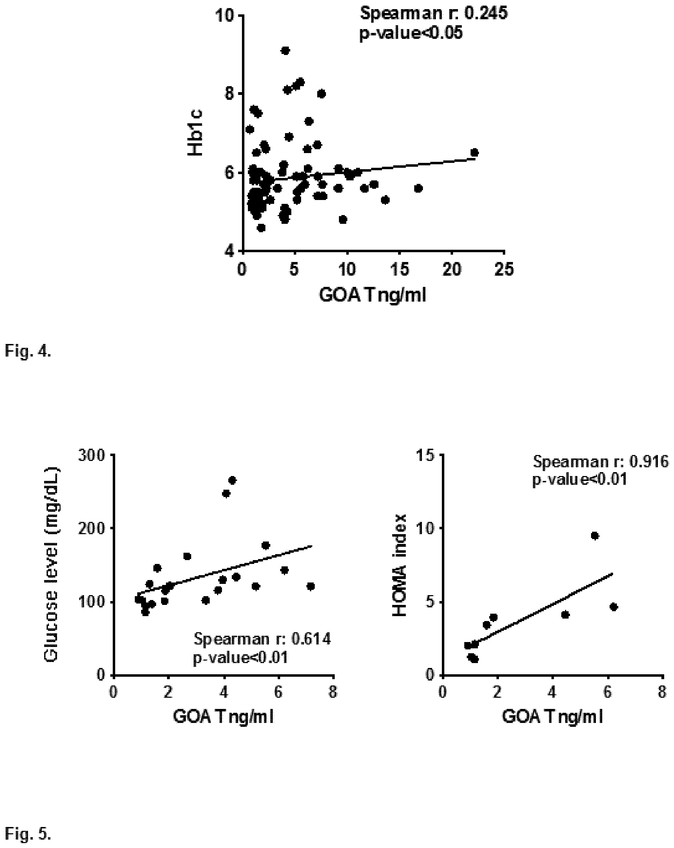

Finally, it must be noted that the plasma GOAT levels detected in patients with prostate cancer correlate with several glucose homeostasis-, diabetes-, and metabolic syndrome-related markers. Specifically, the levels of GOAT are positively correlated with the levels of glycosylated hemoglobin in patients with prostate cancer (Figure 4). Furthermore, they were correlated with glucose levels and with the HOMA-IR index in patients with prostate cancer who have metabolic syndrome (Figure 5).

Therefore, a first aspect of the present disclosure relates to the use of the levels of the products of expression of the gene encoding the ghrelin-O-acyltransferase (GOAT) enzyme, obtained *in vitro* (outside the human or animal body) from a biological sample isolated from a subject, preferably from a human, as a biomarker or indicator for obtaining data that can be used in the diagnosis or detection of prostate cancer.

Preferably, the present disclosure relates to the *in vitro* use of the levels (expressed in terms of amount or concentration) of the ghrelin-O-acyltransferase (GOAT) enzyme or of the mRNA encoding said enzyme, obtained from a biological sample isolated from a subject, preferably from a human, as a biomarker or indicator for obtaining data that can be used in the diagnosis or detection of prostate cancer.

Also preferably, in the present disclosure the levels of the ghrelin-O-acyltransferase (GOAT) enzyme are determined from a sample isolated from blood, serum, a sample isolated from prostate tissue or plasma, and the determination of the mRNA levels is determined from the sample isolated from prostate tissue.

As it is used herein, the term "products of expression of the gene..." refers to the products of transcription and/or translation (RNA and/or protein) of said gene, or to any form resulting from the processing of said transcription or translation products.

An "isolated biological sample" includes, without limitation, cells, tissues, and/or biological fluids from an organism, obtained by means of any method known to one skilled in the art. The biological sample can be a tissue, for example, a biopsy or a fine-needle aspirate, or a fluid sample, such as a blood, plasma, or serum sample. Preferably, the isolated biological samples are selected from the list consisting of blood, plasma, serum, or a prostate tissue biopsy or sample.

As it is used herein, the term "cancer" or "cancerous" refers to any malignant tumor, specifically a malignant prostate tumor.

The determination of the levels of the amount or concentration, preferably in a semi-quantitative or quantitative manner, can be carried out directly or indirectly. Direct measurement refers to measurement of the amount or concentration of the products of expression based on a signal which is obtained directly from the products of expression and is correlated with the number of molecules of said products of expression. Said signal, which can also be referred to as an intensity signal, can be obtained by measuring a value of intensity of a chemical or physical property of said products of expression, for example. Indirect measurement includes measurement obtained from a secondary component or a biological measurement system (for example, the measurement of cell responses, ligands, "labels", or enzymatic reaction products).

As it is used herein, the term "amount" refers, but is not limited, to the absolute or relative amount of the products of expression, as well as to any other value or parameter that is related to the products of expression or can be derived from same. Said values or parameters comprise the values of intensity of the signal obtained from any of the physical or chemical properties of the products of expression obtained by means of direct measurement. Additionally, said values or parameters include all those values or parameters obtained by means of indirect measurement, for example, by means of any of the measurement systems described herein in another part. Therefore, "determination of the expression level" of the genes can be carried out by means of any method for determining the amount of the product of expression of the genes known in the state of the art.

Also preferably in the present disclosure, the detection of the product of expression of the genes is performed by determining the level of mRNA derived from the transcription of said genes, where the GOAT mRNA level can be analyzed, by way of illustration and without limiting the scope of the invention, by means of polymerase chain reaction (PCR) amplification, reverse transcription combined with polymerase chain reaction (RT-PCR), reverse transcription combined with ligase chain reaction (RT-LCR), or any other nucleic acid amplification method; DNA chips prepared with oligonucleotides deposited by means of any mechanism; DNA microarrays prepared with oligonucleotides synthesized *in situ* by means of photolithography or any other mechanism; *in situ* hybridization using specific probes labeled with any labeling method; by means of electrophoresis gels; by means of membrane transfer and hybridization with a specific probe; by means of nuclear magnetic resonance or any other diagnostic imaging technique using paramagnetic nanoparticles or any other type of antibody-functionalized detectable nanoparticles, or by any other means.

The mRNA can be analyzed, for example, but without limitation, in fresh tissue samples, frozen tissue samples, fixed tissue samples, or fixed and paraffin-embedded tissue samples. The use of fixed and paraffin-embedded tissue samples has significant advantages with respect to fresh or frozen tissue samples since they are stable at room temperature, easy to store, and there is a large archive of clinical samples available together with their clinical information and disease monitoring. Therefore, in a preferred embodiment the analyte is extracted from fixed and paraffin-embedded tissue samples.

However, the RNA obtained from fixed and paraffin-embedded tissue samples has usually undergone extensive degradation. While studies by means of microarrays are very sensitive to RNA degradation, the use of RT-PCR, and particularly quantitative RT-PCR (qRT-PCR), has proven to be a technique which offers the best results in light of RNA degradation. Furthermore, expression analysis by means of microarrays is a complex technique that requires sophisticated equipment which is not available in many laboratories. Therefore, in another preferred embodiment detection of the mRNA of the genes is performed by means of the RT-PCR technique; and in a more preferred embodiment, detection is performed by means of the qRT-PCR technique.

Also preferably, in the present disclosure detection of the product of expression of the genes is performed by determining the level of protein derived from the transcription and translation of said genes, where the level of the peptide products of GOAT can be analyzed, by way of illustration and without limiting the scope of the present disclosure, by means of incubation with a specific antibody in an immunoassay. As it is used herein, the term "immunoassay" refers to any analytical technique which is based on the conjugation reaction of an antibody with the obtained sample. Examples of immunoassays known in the state of the art are, for example, but without limitation, immunoblot, enzyme-linked immunosorbent assay (ELISA), radioimmunoassay (RIA), immunohistochemistry, or protein microarrays.

A second aspect of the disclosure relates to a method for obtaining data that can be used for the diagnosis and/or detection of prostate cancer in a subject, preferably human, wherein the method comprises:
a. detecting and quantifying, *in vitro,* in a biological sample isolated from said subject, the amount or concentration of the ghrelin-O-acyltransferase (GOAT) enzyme or of the mRNA encoding said proteins.

Preferably, in the second aspect of the disclosure the isolated biological sample is selected from the list consisting of blood, plasma, serum, or a prostate tissue biopsy or sample.

Also preferably, in the second aspect of the disclosure, the method further comprises:
b. comparing the levels obtained in step (a) with a reference level or with the levels obtained in a control sample obtained from a healthy human subject.

A first aspect of the invention relates to a method for the diagnosis and/or detection of prostate cancer, wherein the method comprises steps (a) - (b) of the second aspect of the disclosure and further comprises:
c. assigning the subjects having increased levels with respect to a reference level to the group of individuals suffering from prostate cancer.

As it is used herein, the term "diagnosis" refers to the capacity to differentiate between individuals suffering from prostate cancer and those who are not. It also refers, without limitation, to the capacity to differentiate between samples originating from patients and sample originating from healthy individuals. This differentiation, as is understood by one skilled in the art, does not seek to be correct for all the analyzed samples. However, it does require a statistically significant amount of the analyzed samples to be correctly classified. The statistically significant amount can be established by one skilled in the art by using different statistical tools, for example, but without limitation, by means of determining confidence intervals, determining the p-value, Student's t-test, or Fisher's discriminant functions. The confidence intervals are preferably at least 90%, at least 95%, at least 97%, at least 98%, or at least 99%. The p-value is preferably less than 0.1, 0.05, 0.01, 0.005, or 0.0001. The present invention preferably allows correctly detecting the disease in a differential manner in at least 60%, in at least 70%, in at least 80%, or in at least 90% of the subjects of a specific group or analyzed population.

The detection of GOAT (of both the mRNA and the actual protein) in a biological sample isolated from a subject in a proportion greater than the reference amount is indicative of said individual suffering from prostate cancer. In particular, the levels obtained in a control sample obtained from a healthy human subject that are increased by at least 1.5-fold, preferably 2-fold, are indicative of said individual suffering from prostate cancer.

A first alternative aspect of the invention therefore relates to a method for the diagnosis and/or detection of prostate cancer, wherein the method comprises steps (a) - (b) of the second aspect of the invention and further comprises:
c. assigning the subjects having levels increased by at least 1.5-fold, preferably by 2-fold, with respect to the levels obtained in a control sample obtained from a healthy human subject, to the group of individuals suffering from prostate cancer.

The term "subject" does not seek to be limiting in any aspect, where said subject can be of any age and have any physical condition.

Another aspect of the disclosure, i.e., the third aspect, relates to a kit comprising the means suitable for carrying out the methods of the invention. Preferably, in the third aspect of the disclosure, said kit comprises the means for detecting the products of expression (particularly mRNA and protein) derived from of the gene generating the GOAT.

A particular aspect of the disclosure relates to a kit or device suitable for carrying out a PCR reaction, preferably RT-PCR, qRT-PCR, or qPCR, wherein the kit comprises primers consisting essentially of sequences SEQ ID NO 1 and SEQ ID NO 2. Said kit can further comprise all those reagents required for carrying out said PCR reactions. In this sense, the kit can further include, without any type of limitation, the use of buffers, enzymes, polymerase enzymes, cofactors to obtain optimum enzyme activity, agents to prevent contamination, etc. On the other hand, the kit can include all the supports and containers required for putting it in use and for optimizing same. The kit can further contain other molecules, genes, proteins, or probes of interest, which serve as positive and negative controls. The kit preferably further comprises instructions for carrying out the fourth aspect of the invention. The kit preferably comprises, in addition to the indicated primers, at least one or any combination of the following reagents: DNA polymerase, dNTPs, MgCl₂, markers, stabilizers, DNAses, and reverse transcriptase (RT).

A second aspect of the invention relates to the *in vitro* use of a kit or device in the methods of the first aspects of the invention, which comprises specific primers for the determination of the expression levels of GOAT by means of PCR for obtaining data that can be used for the diagnosis and/or detection of prostate cancer in a human subject based on a biological sample isolated from said subject. Said kit is preferably defined according to the third aspect of the disclosure. Said biological sample is preferably selected from the list consisting of blood, plasma, serum, and samples isolated from prostate tissue.

A third aspect of the invention relates to the *in vitro* use of a kit or device in the methods of the first aspects of the invention, which comprises antibodies or fragments thereof that are specific against the GOAT protein for obtaining data that can be used for the diagnosis and/or detection of prostate cancer in a human subject based on a biological sample isolated from said subject. Said biological sample is preferably a prostate tissue biopsy. The use of the third aspect of the invention is preferably carried out by means of an immunoassay, preferably by means of an ELISA, more preferably by means of the *in vitro* use of a commercial ELISA (Human Ghrelin-O-Acyltransferase (GOAT) ELISA Kit; MBS2019923, MyBioSource, San Diego, CA), following the manufacturer's instructions. In another preferred embodiment, the immunoassay is an immunoblot or Western blot. To carry out an immunoblot or Western blot, a protein extract is obtained from a biological sample isolated from a subject and the proteins are separated in a support medium capable of retaining said proteins by means of electrophoresis. Once separated, the proteins are transferred to a different support where they can be detected by means of using specific antibodies which recognize the peptide products of GOAT, In1-Ghrelin, and/or truncated ghrelin receptor (GHS-R1b).

In another preferred embodiment, the immunoassay is an immunohistochemistry (IHC). Immunohistochemistry techniques allow the identification of characteristic antigenic determinants in tissue or cytological samples. Analysis by means of immunohistochemistry is performed on sections of tissue, already frozen or included in paraffin, originating from a biological sample isolated from a subject. These sections are hybridized with a monoclonal or polyclonal antibody which recognizes the peptide products of GOAT, In1-Ghrelin, and/or truncated ghrelin receptor (GHS-R1b).

Tissue microarray is among the immunohistochemistry techniques that can be used for obtaining expression profiles. Tissue microarray (TMA) is considered a powerful tool today for the simultaneous mass analysis of the cancer molecular profile of various tissue samples, providing the possibility of studying new or already existing markers in a large scale. In turn, TMA preserves the original tissue samples the amount of which is reduced, and which deteriorate as a result of frequent use in conventional methods.

As it is used herein, the term "antibody" refers to immunoglobulin molecules and immunologically active portions of immunoglobulin molecules, i.e., molecules containing an antigen-binding site that binds specifically (immunoreacts) with any of the peptide products of GOAT. Examples of immunologically active immunoglobulin molecule fragments include F(ab) and F(ab')2 fragments which can be generated by treating the antibody with an enzyme such as pepsin. The antibodies can be polyclonal antibodies (they typically include different antibodies targeting different determinants or epitopes) or monoclonal antibodies (targeting a single determinant in the antigen). The monoclonal antibody can be altered biochemically, by means of genetic manipulation, or it can be synthetic, the antibody possibly lacking, entirely or in parts, portions that are not required for the recognition of the peptide products of GOAT and being substituted by others conferring additional advantageous properties to the antibody. The antibody can also be recombinant, chimeric, humanized, synthetic, or a combination of any of the foregoing. A "recombinant antibody or polypeptide" (rAB) is an antibody that has been produced in a host cell which has been transformed or transfected with the nucleic acid that encodes the polypeptide or produces the polypeptide as a result of homologous recombination.

Throughout the description and claims the word "comprises" and variants thereof do not seek to exclude other technical features, additions, components, or steps. For those skilled in the art, other objects, advantages, and features of the invention will be inferred in part from the description and in part from putting the invention into practice.

The following examples and drawings are provided by way of illustration and do not seek to limit the present invention.

### EXAMPLES

### Example 1. Materials and methods

### 1.1. Patients and samples

The prostate biopsy samples [both tumor samples (n=37) and normal samples (n=10)] were obtained from Hospital Universitario Reina Sofia (Cordoba) after proper evaluation by an expert pathologist and under the approval of the Ethics Committee of Universidad de Córdoba and Hospital Reina Sofia (Cordoba). It is a cohort of patients suspected of having prostate cancer on whom biopsies were performed between 2012 and 2014 to confirm said suspicion. Blood and urine samples were also collected from said patients during biopsy. All patients signed an informed consent before taking part in the study.

### 1.2. RNA isolation and reverse transcription (RT)

Nucleic acids were isolated from the biopsied tissues by means of using a commercial kit (AllPrep DNA/RNA Mini Kit, Qiagen; Barcelona, Spain) according to the manufacturer's instructions, and they were treated with DNase. The amount of recovered total RNA was determined by means of using the Nanodrop 2000 spectrophotometer (Thermo Scientific, Wilmington, NC, USA). Reverse transcription was carried out with 1 µg of total RNA using the First-Strand Synthesis cDNA kit (Fermentas, Hanover, MD, USA).

### 1.3. Determination of gene expression by means of real-time quantitative PCR (qPCR)

The determination of the expression levels of GOAT was performed by means of qPCR using primers specific for this transcript (Sn (SEQ ID NO 1): TTGCTCTTTTTCCCTGCTCTC and As (SEQ ID NO 2): ACTGCCACGTTTAGGCATTCT). The primers were selected, the specificity was verified, and the efficiency was confirmed in a manner similar to that described previously for other genes [17, 25]. The final volume of the PCR reaction was 20 µl including 100 ng of sample and 10 µl of IQ™ SYBR Green Supermix (Biorad). The PCR program consisted of 40 cycles at 95°C for 20 s, 60°C for 20 s, and 55°C for 30 s. Furthermore, a DNA-free control was run in each plate to monitor possible exogenous contaminations. In all the cases, amplification was carried out with the Mx3000 qPCR system (Agilent, Madrid). To confirm that only one band of the expected size was amplified, the products were run in an agarose gel stained with ethidium bromide. This band was purified and then sequenced to confirm that it corresponded with the expected sequence.

### 1.4. Determination of the levels of GOAT protein

The plasma or urine GOAT concentration was determined by means of using a commercial ELISA (Human Ghrelin-O-Acyltransferase (GOAT) ELISA Kit; MBS2019923, MyBioSource, San Diego, CA) following the manufacturer's instructions. Information relating to specificity, detectability, and assay reproducibility are available on the company's webpage.

### Example 2. RESULTS

### 2.1. Expression of GOAT mRNA in prostate biopsy samples

The expression levels of GOAT, measured by real-time quantitative PCR (qPCR), are significantly increased in samples originating from the biopsies of patients with prostate cancer in comparison with samples originating from the biopsies of healthy patients (in which the biopsy was negative for the presence of prostate cancer) (Figure 1, left panel). In fact, the expression levels of GOAT in the biopsies can significantly distinguish patients with prostate cancer from healthy individuals with a high sensitivity and specificity (ROC curve; Figure 1, right panel).

### 2.2. Levels of GOAT in biofluids from patients suspected of having prostate cancer

Furthermore, the levels of GOAT protein in biofluids (plasma and urine) from said patients were determined. These analyses revealed that GOAT is not found at detectable levels in urine; whereas it is found at considerable (detectable) levels in plasma samples. Specifically, the GOAT enzyme is also found at high levels in the plasma of patients with prostate cancer compared with the plasma of healthy control patients (Figure 2, left panel). Similarly, plasma GOAT levels can distinguish patients with prostate cancer from healthy individuals with a high sensitivity and specificity (ROC curve; Figure 2, right panel). Therefore, a serum GOAT value of 1.212 ng/mL shows a sensitivity of 81% and a specificity of 66% for distinguishing patients with prostate cancer from those without.

Furthermore, the plasma GOAT levels detected in patients with prostate cancer correlated positively with the total PSA levels (TPSA) and negatively with free PSA (FPSA) levels (Figure 3). Similarly, the plasma GOAT levels detected in patients with prostate cancer also correlated positively with other two tumor markers, such as ca153 and cifra_211 (Figure 3).

Finally, it must be noted that the plasma GOAT levels detected in patients with prostate cancer correlate with several glucose homeostasis dysregulation-, diabetes-, and metabolic syndrome-related markers. Specifically, the levels of GOAT are positively correlated with the levels of glycosylated hemoglobin in patients with prostate cancer (Figure 4). Furthermore, they were correlated with glucose levels and with the HOMA-IR index in patients with prostate cancer who have metabolic syndrome (Figure 5).

### BIBLIOGRAPHY

1. Kojima, M., et a/., Ghrelin is a growth-hormone-releasing acylated peptide from stomach. Nature, 1999. 402(6762): p. 656-60.
2. Lago, F., et a/., Ghrelin, the same peptide for different functions: player or bystander? Vitam Horm, 2005. 71: p. 405-32.
3. Leite-Moreira, A.F., A. Rocha-Sousa, and T. Henriques-Coelho, Cardiac, skeletal, and smooth muscle regulation by ghrelin. Vitam Horm, 2008. 77: p. 207-38.
4. Leite-Moreira, A.F. and J.B. Soares, Physiological, pathological and potential therapeutic roles of ghrelin. Drug Discov Today, 2007. 12(7-8): p. 276-88.
5. Leontiou, C.A., G. Franchi, and M. Korbonits, Ghrelin in neuroendocrine organs and tumours. Pituitary, 2007. 10(3): p. 213-25.
6. Horvath, T.L., et a/., Minireview: ghrelin and the regulation of energy balance--a hypothalamic perspective. Endocrinology, 2001. 142(10): p. 4163-9.
7. Tschop, M., D.L. Smiley, and M.L. Heiman, Ghrelin induces adiposity in rodents. Nature, 2000. 407(6806): p. 908-13.
8. van der Lely, A.J., et a/., Biological, physiological, pathophysiological, and pharmacological aspects of ghrelin. Endocr Rev, 2004. 25(3): p. 426-57.
9. Gutierrez, J.A., et a/., Ghrelin octanoylation mediated by an orphan lipid transferase. Proc Natl Acad Sci USA, 2008. 105(17): p. 6320-5.
10. Yang, J., et al., Identification of the acyltransferase that octanoylates ghrelin, an appetite-stimulating peptide hormone. Cell, 2008. 132(3): p. 387-96.
11. Chang, S.C. and A.I. Magee, Acyltransferases for secreted signalling proteins (Review). Mol Membr Biol, 2009. 26(1): p. 104-13.
12. Lim, C.T., B. Kola, and M. Korbonits, The ghre/in/GOAT/GHS-R system and energy metabolism. Rev Endocr Metab Disord, 2011. 12(3): p. 173-86.
13. Muller, T.D., et a/., Genetic variation of the ghrelin activator gene ghrelin O-acyltransferase (GOAT) is associated with anorexia nervosa. J Psychiatr Res, 2011. 45(5): p. 706-11.
14. Taylor, M.S., et a/., Architectural Organization of the Metabolic Regulatory Enzyme Ghrelin-O-Acyltransferase. J Biol Chem, 2013.
15. Lim, C.T., et a/., The expression of ghrelin O-acyltransferase (GOAT) in human tissues. Endocr J, 2011. 58(8): p. 707-10.
16. Sakata, I., et a/., Co-Localization of Ghrelin O-Acyltransferase (GOAT) and Ghrelin in Gastric Mucosal Cells. Am J Physiol Endocrinol Metab, 2009. 297(1):E134-41**.**
17. Gahete, M.D., et a/., A novel human ghrelin variant (In1-ghrelin) and ghrelin-O-acyltransferase are overexpressed in breast cancer: potential pathophysiological relevance. PLoS One, 2011. 6(8): p. e23302.
18. Papotti, M., et a/., Ghrelin-producing endocrine tumors of the stomach and intestine. J Clin Endocrinol Metab, 2001. 86(10): p. 5052-9.
19. Rindi, G., et a/., Ghrelin expression in gut endocrine growths. Histochem Cell Biol, 2002. 117(6): p. 521-5.
20. Volante, M., et a/., Expression of ghrelin and of the GH secretagogue receptor by pancreatic islet cells and related endocrine tumors. J Clin Endocrinol Metab, 2002. 87(3): p. 1300-8.
21. Inhoff, T., et a/., Desacyl ghrelin inhibits the orexigenic effect of peripherally injected ghrelin in rats. Peptides, 2008. 29(12): p. 2159-68.
22. Ekeblad, S., et al., Co-expression of ghrelin and its receptor in pancreatic endocrine tumours. Clin Endocrinol (Oxf), 2007. 66(1): p. 115-22.
23. Chopin, L.K., et a/., The ghrelin axis--does it have an appetite for cancer progression? Endocr Rev, 2012. 33(6): p. 849-91.
24. Seim, I., et a/., Ghrelin O-acyltransferase (GOAT) is expressed in prostate cancer tissues and cell lines and expression is differentially regulated in vitro by ghrelin. Reprod Biol Endocrinol, 2013. 11: p. 70.
25. Ibanez-Costa, A., et a/., In1-ghrelin splicing variant is overexpressed in pituitary adenomas and increases their aggressive features. Sci Rep, 2015. 5: p. 8714.

## Claims

1. A method for the diagnosis of prostate cancer in a human subject, wherein the method comprises:
a. detecting and quantifying, *in vitro,* the expression of ghrelin-O-acyltransferase (GOAT) enzyme mRNA in a prostate tissue biopsy or sample isolated from said subject or the amount of the GOAT protein in a blood, plasma, or serum sample isolated from said subject;
b. comparing the levels obtained in step (a) with a reference level or with the levels obtained in a control sample obtained from a healthy human subject; and
c. assigning the subjects having increased levels with respect to a reference level to the group of individuals suffering from prostate cancer.

2. The method according to claim 1, wherein step c) comprises:
c. assigning the subjects having levels increased by at least 2-fold, with respect to the levels obtained in a control sample obtained from a healthy human subject, to the group of individuals suffering from prostate cancer.

3. *In vitro* use of a kit or device in the method of any of claims 1 to 2, wherein the kit or device comprises specific primers for the determination of the expression levels of GOAT mRNA by means of PCR .

4. The use according to claim 3, wherein the kit or device comprises primers consisting essentially of sequences SEQ ID NO 1 and SEQ ID NO 2.

5. The use according to claim 4, wherein the kit or device further comprises at least one or any combination of the following reagents: DNA polymerase, dNTPs, MgCl₂, markers, stabilizers, DNAses, and the reverse transcriptase (RT) enzyme.

6. *In vitro* use of a kit or device in the method of any of claims 1 to 2, wherein the kit or device comprises antibodies or fragments thereof that are specific against the GOAT protein.

## Patentansprüche

1. Verfahren zur Diagnose von Prostatakrebs in einem menschlichen Individuum, wobei das Verfahren Folgendes umfasst:
a. das, *in-vitro,* Detektieren und Quantifizieren der Expression von mRNA des Ghrelin-O-Acyltransferase (GOAT)-Enzyms in einer Prostatagewebebiopsie oder -probe isoliert aus dem genannten Individuum oder der Menge vom GOAT-Protein in einer Blut-, Plasma- oder Serumprobe isoliert aus dem genannten Individuum;
b. das Vergleichen der in Schritt (a) erhaltenen Spiegel mit einem Referenzspiegel oder mit den Spiegeln erhalten in einer Kontrollprobe, welche aus einem gesunden menschlichen Individuum erhalten wird; und
c. das Zuordnen der Individuen, welche erhöhte Spiegel in Bezug auf einen Referenzspiegel aufweisen, zur Gruppe von Individuen, welche unter Prostatakrebs leiden.

2. Verfahren nach Anspruch 1, wobei Schritt c) Folgendes umfasst:
c. das Zuordnen der Individuen, welche mindestens 2-mal erhöhte Spiegel in Bezug auf die Spiegel erhalten in einer Kontrollprobe, welche aus einem gesunden menschlichen Individuum erhalten wird, aufweisen, zur Gruppe von Individuen, welche unter Prostatakrebs leiden.

3. *In-vitro*-Verwendung eines Kits oder einer Vorrichtung im Verfahren nach einem der Ansprüche 1 bis 2, wobei das Kit oder die Vorrichtung spezifische Primer zur Bestimmung der Expressionniveaus von GOATmRNA mittels PCR umfasst.

4. Verwendung nach Anspruch 3, wobei das Kit oder die Vorrichtung Primer umfasst, welche im Wesentlichen aus den Sequenzen SEQ ID NO 1 und SEQ ID NO 2 bestehen.

5. Verwendung nach Anspruch 4, wobei das Kit oder die Vorrichtung zusätzlich mindestens eine oder jegliche Kombination der folgenden Reagenzien umfasst: DNA-Polymerase, dNTP, MgCl₂, Marker, Stabilisatoren, DNAsen und das Reverse-Transkriptase (RT)-Enzym.

6. *In-vitro*-Verwendung eines Kits oder einer Vorrichtung im Verfahren nach einem der Ansprüche 1 bis 2, wobei das Kit oder die Vorrichtung Antikörper oder Fragmente derselben umfasst, welche spezifisch gegen das GOAT-Protein sind.

## Revendications

1. Procédé pour le diagnostic du cancer de la prostate chez un sujet humain, dans lequel le procédé comprend :
a. la détection et la quantification, *in vitro*, de l'expression d'ARNm de l'enzyme ghréline-O-acyltransférase (GOAT) dans une biopsie ou un échantillon de tissu de prostate isolé dudit sujet ou la quantité de la protéine GOAT dans un échantillon de sang, de plasma ou de sérum isolé dudit sujet ;
b. la comparaison des niveaux obtenus à l'étape (a) avec un niveau de référence ou avec les niveaux obtenus dans un échantillon de contrôle obtenu d'un sujet humain sain ; et
c. l'assignation des sujets présentant des niveaux accrus par rapport à un niveau de référence au groupe d'individus atteints de cancer de la prostate.

2. Procédé selon la revendication 1, dans lequel l'étape c) comprend :
c. l'assignation des sujets présentant des niveaux accrus au moins 2 fois, par rapport aux niveaux obtenus dans un échantillon de contrôle obtenu d'un sujet humain sain, au groupe d'individus atteints de cancer de la prostate.

3. Utilisation *in vitro* d'une trousse ou d'un dispositif dans le procédé selon l'une quelconque des revendications 1 à 2, dans laquelle la trousse ou le dispositif comprend des amorces spécifiques pour la détermination des niveaux d'expression de l'ARNm de GOAT au moyen de PCR.

4. Utilisation selon la revendication 3, dans laquelle la trousse ou le dispositif comprend des amorces constituées essentiellement des séquences SEQ ID NO 1 et SEQ ID NO 2.

5. Utilisation selon la revendication 4, dans laquelle la trousse ou le dispositif comprend en outre au moins un ou une combinaison quelconque des réactifs suivants : ADN polymérase, dNTP, MgCl₂, marqueurs, stabilisateurs, ADNases et l'enzyme transcriptase inverse (RT).

6. Utilisation *in vitro* d'une trousse ou d'un dispositif dans le procédé selon l'une quelconque des revendications 1 à 2, dans laquelle la trousse ou le dispositif comprend des anticorps ou des fragments de ceux-ci qui sont spécifiques contre la protéine GOAT.
